# EUROPEAN PATENT APPLICATION

(11) **EP 1 130 089 A1**
(43) Date of publication of application: **05.09.2001**
(21) Application number: 00103242.4
(22) Date of filing: 17.02.2000
(51) Int. Cl.: C12N 7/04, A61K 39/21, C07K 14/705, C07K 14/715, C07K 14/16

(54) **Composition comprising membrane virus subviral target and fusion particles and vaccine comprising said composition**

(71) Applicant: Deutsches Krebsforschungszentrum Stiftung des öffentlichen Rechts, 69120 Heidelberg (DE)
(72) Inventor: Bosch, Valerie, Dr., 69245 Bammental (DE); Sparacio, Sandra, 69214 Eppelheim (DE); Zeilfelder, Udo, 68259 Mannheim (DE); Pfeiffer, Tanya, 69221 Dossenheim (DE); Henzler, Tanya, 69126 Heidelberg (DE)
(74) Representative: Schüssler, Andrea, Dr.

(57) **Abstract**

Described is a composition of membrane virus subviral particles, preferably retrovirus-like, more preferably HIV-like subparticles, comprising (a) an env-defective, at least one cellular receptor and at least one coreceptor containing membrane virus target particle encoded by an env-defective membrane virus particle encoding vector construct, at least one cellular receptor encoding vector(s) and at least one coreceptor encoding vector(s) and (b) a membrane virus fusion particle encoded by an env-defective membrane virus particle encoding vector construct and an env-encoding vector, wherein said composition of membrane virus subviral particles is capable of inter-membrane virus particle membrane fusion resulting in the formation of membrane-virus particles. Also described is a vaccine comprising the composition of the present invention.

## Description

The present invention relates to a composition of membrane virus subviral particles, comprising (a) an env-defective, at least one cellular receptor and at least one coreceptor containing membrane virus target particle encoded by an env-defective membrane virus particle-encoding vector construct, at least one cellular receptor encoding vector(s) and at least one coreceptor encoding vector(s) and (b) a membrane virus fusion particle encoded by an env-defective membrane virus particle-encoding vector construct and an env-encoding vector, wherein said composition of membrane virus subviral particles is capable of inter-membrane virus particle membrane fusion resulting in the formation of fused (fusing) membrane virus particles. In a preferred embodiment said particles are retrovirus-like, e.g. HIV-like, particles. Additionally, the present invention relates to a vaccine comprising the composition of the present invention.

Due to the expanding epidemic of human immunodeficiency virus (HIV) infections, the need for an effective HIV vaccine is urgent. However progress toward this goal is so far rather limited inter alia due to the fact that (a) the use of recombinant forms of the surface gp120 subunit of the HIV envelope protein does not lead to the generation of antibodies which are capable of cross-neutralizing the virus and (b) many pathogenic membrane viruses, e.g. HIV and hepatitis C virus, undergo sequence variability. Actually, there has been so far no solution to the latter problem. A strategy for the use of fixed preparations of cells undergoing membrane fusion mediated by the human immunodeficiency virus glycoprotein and the cellular receptor complex consisting of CD4 and CCR5 has been published and tested in a mouse model system (1). However, in this situation the potential vaccine is not molecularly defined and the relevant immunogen is contaminated with a very large excess of undefined cellular materials and, thus, not useful for the preparation of vaccine for the application to humans. To summarize, the disadvantage (and problem) of current candidate vaccines to many pathogenic membrane viruses is that they do not provide protection to different heterologous strains and/or are not molecularly defined.

Thus, the technical problem underlying the present invention is to provide a molecularly defined vaccine for the prevention of infection by pathogenic membrane viruses, particularly pathogenic retroviruses, including viruses undergoing sequence variations.

The solution to the above technical problem has been achieved by providing the embodiments characterized in the claims.

In the present invention two different types of molecularly defined membrane virus subviral particles with incorporated viral and cellular receptors of pathogenic membrane viruses were fixed in the process of inter-particle membrane fusion. It could be shown that specific inter-membrane virus particle fusion can occur, that fused (fusing) membrane virus particles are infectious and that these have the host-range of both fusion partners. Purified preparations of these fusing particles can be applied as effective vaccine to induce protective immune responses against the respective disease. The process of membrane fusion, induced by viral glycoproteins and their specific cellular receptors involves complex and sequential conformational changes on the viral glycoprotein. These conformational changes are essential for function and are, thus, functionally conserved. During this process new conformational epitopes are exposed on the viral glycoprotein and an immune response against these will be induced in the vaccinated organism. The results obtained demonstrate the usefulness of a vaccine strategy employing fixed preparations of molecularly well-defined virus particles in the process of membrane fusion, e.g. as regards vaccination against HIV fixed preparations of well-defined fusing CD4/coreceptor target particles and (Wt)HIV-Env fusion particles. Moreover, the response obtained by this strategy of vaccination is protective against different strains of the respective virus since the exposed conformational epitopes are functionally conserved. The strategy shown for HIV in the Examples below is applicable to the glycoproteins of other pathogenic membrane viruses and their cellular receptors (e.g. hepatitis C virus).

Thus, in one aspect, the present invention relates to a composition of membrane virus subviral particles, comprising (a) an env-defective, at least one cellular receptor and at least one coreceptor containing membrane virus target particle encoded by an env-defective membrane virus particle-encoding vector construct, at least one cellular receptor encoding vector(s) and at least one coreceptor encoding vector(s) and (b) a membrane virus fusion particle encoded by an env-defective membrane virus particle-encoding vector construct and an env-encoding vector, wherein said composition of membrane virus subviral particles is capable of inter-membrane virus particle membrane fusion resulting in the formation of fused (fusing) membrane virus particles. These subviral particles are obtainable by cotransfection of a mammalian host cell with the vectors of (a) and (b), respectively.

As used herein, the term "membrane virus subviral particle" relates to a particle encoded by an expression vector derived from a membrane virus genome but lacking or having mutated one or more genes required for infectivity. Examples of pathogenic membrane viruses are retroviruses, e.g. HIV and Hepatitis C virus, Yellow fever virus, tick borne encephalitis virus, Rabies virus, Ebola virus, Marburg virus, Bornavirus, Influenza virus, Herpes virus, Hepatitis B virus etc. The target particle can, in principle, be generated by cotransfecting suitable mammalian host cells, e.g. 293T cells, with an Env-defective particle-encoding vector construct, e.g. the construct described in Example 1, below, and expression vectors for the cellular receptor, e.g. as regards HIV CD4, and a coreceptor, e.g. as regards HIV the CC chemokine receptor 5 (CCR5) or the CXC chemokine receptor 4 (CXCR4). The fusion particle can, in principle, be generated by cotransfecting suitable mammalian host cells, e.g. 293T cells with an Env-defective particle-encoding vector construct e.g. the construct described in Example 1, below, and expression vectors encoding the membrane virus Env, e.g. HIV-Env.

As used herein, the term "env" denotes viral receptor glycoproteins of membrane viruses capable of specific interaction with cellular receptor and cellular coreceptor.

As used herein, the term "env-defective membrane virus particle-encoding vector construct" relates to an expression vector encoding genes required for membrane virus particle production and not encoding the env gene product. The required genes are those needed for the expression of the structural proteins which together comprise the membrane virus particle. In the case of HIV, this would be the gag gene, which in its wild-type form requires the additional presence of the rev responsive element (RRE) and coexpression of the rev gene. Env-defectiveness can be achieved e.g. by the absence of the env gene in the particle-encoding vector or by employing particle-encoding vectors in which the env gene has been mutated such that no or only a non-functional protein fragment is synthesised. The env inactivation mutation can be achieved e.g. by a deletion or insertion of nucleotides at the beginning of the gene leading to a frame-shift in translation. This can be done by standard in vitro mutagenesis procedures. The Env-defective membrane virus vector construct can encode further genes (e.g. in the case of HIV in addition to gag and rev, also pol and tat) and can include further cis-elements (e.g. in the case of HIV LTR etc). These would be relevant for testing the extent of particle fusion in infectivity/transduction assays. Vectors which in the case of HIV lead to "env-defective membrane virus vector particles" are e.g. described below in the Examples, or are pKExHIVΔenv3 (11; Wilk et al; Virology 218 (1996), 269-274), pK-R-gpII and pK-R-gpIIa (Mergener et al; Virology 186, (1992) 25-29).

As used herein, the term "cellular receptor" relates to any form of the receptor which can be incorporated into the membrane virus particles and which is capable of binding the membrane virus envelope and facilitating the subsequent interaction with one or more of coreceptor molecules. As used herein, the term "coreceptor" relates to any form of the coreceptor which can be incorporated into the membrane virus particles and which is capable of interacting with the membrane virus envelope. The above terms comprise native receptors/coreceptors as well as variants containing mutations or deletions which do not substantially affect the biological activity thereof. Such mutations include substitutions of one or more amino acids, particularly by homologues thereof, as well as additions of one or more amino acids, especially at the N or C termini. Deletions include deletions from the N or C termini. Substitutions by both naturally-occurring and synthetic amino acids are possible. Also included in this definition are receptor/coreceptor variants modified by chemical modification or enzymatic modification. Further, fragments of the receptors/coreceptors are included within the definition of the term "cellular receptor" or "coreceptor" as long as they are functional. For the manipulation in prokaryotic cells by means of genetic engineering the DNA sequences described above or parts of these sequences can be introduced into plasmids allowing a mutagenesis or a modification of a sequence by recombination of DNA sequences. By means of conventional methods (cf. Sambrook et al., 1989, Molecular Cloning: A Laboratory Manual, 2^{nd} edition, Cold Spring Harbor Laboratory Press, NY, USA) bases can be exchanged and natural or synthetic sequences can be added. In order to link the DNA fragments with each other adapters or linkers can be added to the fragments. Furthermore, manipulations can be performed that provide suitable cleavage sites or that remove superfluous DNA or cleavage sites. If insertions, deletions or substitutions are possible, in vitro mutagenesis, primer repair, restriction or ligation can be performed. As analysis method usually sequence analysis, restriction analysis and other biochemical or molecular biological methods are used.

Examples of cellular receptors are those for retroviruses, e.g. CD4 (for HIV), Tva (for Rous sarcoma virus, subgroup A), MCAT-1 (for ecotropic murine leukemia virus) and several others (summarised in Virology, Chapter 58, eds. Fields, Knipe, Howley, Lippencott Raven Publishers, (1996)). Further examples of cellular receptors are sialic acid containing glycoproteins or glycolipids for Influenza virus and CD81 for Hepatitis C virus (Pileri et al., Science 282, (1998), 938-941). Additionally, examples of coreceptors are CCR5, CXCR4, CCR-2B, CCR-3, CCR-8, CCR-9, CXCR-1 and CX3CR-1 (all for HIV) (Berger et al., Ann. Rev. Immunol. 17, (1999), 657-700).

As used herein, the term "mammalian host cell" comprises any mammalian host cell which can be transfected with the vectors of the invention, allows gene expression and particle formation. Useful mammalian host cells are well known to the person skilled in the art and comprise cells such as CHO, COS, 293T, 293, HeLa, BJAB, HaCAT and Bowes melanoma cells.

The construction of the vectors needed for obtaining the composition of the present invention, i.e. the env-defective membrane virus particle-encoding vector construct of (a), the cellular receptor(s) encoding vector(s), the coreceptor(s) encoding vector(s), the env-defective membrane virus particle-encoding vector construct of (b) and the env-encoding vector can be carried out according to conventional methods (cf. Sambrook et al., 1989, Molecular Cloning: A Laboratory Manual, 2^{nd} edition, Cold Spring Harbor Laboratory Press, NY, USA). Suitable vectors allowing amplification in prokarytic and eukaryotic expression systems including the mammalian host cells described above are, e.g those described below in the Examples, or are pKEx2XR and pKEx2XL (Rittner et al., Methods in Molecular and Cellular Biology 2, (1991), 176-181). These vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Representative examples of appropriate hosts include, but are not limited to, bacterial cells, such as E. coli, Streptomyces and Salmonella typhimurium cells; fungal cells, such as yeast cells; insect cells such as Drosophila S2 and Spodoptera Sf9 cells; animal; and plant cells. The DNA sequences inserted into said vectors and encoding the various proteins of the membrane virus subviral particles, receptors and coreceptors can be obtained from known sources. For instance, the HIV sequences and the sequences for CD4 and coreceptors are obtainable through the AIDS Research and Reference Reagent Program. NIH, NIAID. The required sequences are those genes usable for particle formation as described above, as well as those genes encoding functional forms of the respective viral glycoprotein, in the first instance HIV glycoprotein of different subgroups and sequences, and the genes encoding the appropriate functional cellular receptor and coreceptor molecules. The DNA sequences encoding the membrane virus or cellular proteins may be amplified by PCR amplification using suitably designed primers. The composition of the present invention is also obtainable using vector constructs wherein two or more of the inserted DNA sequences discussed above are not contained in separate vectors but instead in a single vector as long as the composition is effective as a vaccine, e.g. the DNA sequences encoding the cellular receptor or the coreceptor can be inserted into the same vector.

The DNA sequences encoding the above proteins may be driven off any appropriate promoter, such as the phage lambda PL promoter, the E. coli lac, trp, phoA and tac promoters, the SV40 early and late promoters and promoters of membrane virus, e.g. retroviral LTRs, to name a few. Other suitable promoters will be known to the skilled artisan. The expression constructs will further contain sites for transcription initiation, termination, and, if necessary in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

Introduction of the above described vectors into the host cell can be effected by well known methods, e.g. calcium phosphate transfection, DEAE-dextran mediated transfection, cationic lipid-mediated transfection, electroporation, transduction, infection, or other methods. Such methods are described in many standard laboratory manuals.

In a preferred embodiment, the membrane virus subviral particles of the composition of the present invention are retrovirus-like subviral particles, more preferably HIV-like subvirus particles in which the cellular receptor is CD4 and the coreceptor CXCR4.

In a further preferred embodiment of the composition of the present invention CD4 is GPI-anchored CD4. GPI-anchor is a modification of the C-terminus of CD4 in which a phosphatidyl inositol moiety is linked through glucosamine and mannose to a phosphoryl ethanolamine residue that is linked to the C-terminal amino acid of CD4 by its amino group. Such a GPI-anchor can be found on the CD55 molecule and transferred to CD4 by standard fusion mutagenesis procedures (Ho et al., Gene 77, (1989), 51-59; Horton et al., Gene 77, (1989), 61-68).

In a more preferred embodiment of the composition of the present invention the env-encoding vector for the membrane virus fusion particle of (b) contains a frame-shift mutation in the 5'-region of the env-gene and a deletion in the packaging signal. Reference is made to the above explanations on page 3, para. 3.

In a further preferred embodiment of the composition of HIV-like subviral particles the gene encoding nef (factor negatively controlling HIV replication in vitro and an vivo and downregulating CD4) in the env-defective membrane virus particle-encoding vector construct for the target particle of (a) is inactivated, preferably by deletion or by replacement by another gene, e.g. a gene the expression of which allows to monitor the infectivity of the fused (fusing) particles in the host cell, e.g. the luciferase encoding gene described in the Examples, below, the green fluorescence protein (GFP) encoding gene or the β-galactosidase encoding gene.

The present invention also relates to a method of producing a membrane virus target particle or a membrane virus fusion particle, comprising (a) cotransfecting a mammalian host cell with the vectors as desribed above for the target particle or fusion particle and cultivating the host cells under conditions such that the proteins encoded by said vectors are expressed and (b) recovering the membrane virus target particle or membrane virus fusion particle from the culture supernatant. The cotransfection and cultivation of the host cells can be carried out according to standard procedures. Appropriate culture mediums and conditions for the above-described host cells are known in the art. The subparticles can be recovered (and purified from) recombinant cell cultures by well-known methods including ultra-centrifugation, ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. This method will allow the production of sufficient amounts of the desired subparticles for use in medical treatments.

Finally, the present invention also relates to a vaccine against membrane virus infections, preferably a retroviral infection, more preferably an HIV infection, comprising a therapeutically effective amount of the composition of the invention described above and the use of the composition of the present invention for the preparation of a prophylactical or therapeutical vaccine, respectively. For the preparation of the vaccine the purified particles may be combined with any suitable adjuvant such as ISCOMS, alum, Freunds Incomplete or Complete Adjuvant, Quil A and other saponins or any other adjuvant as described in the literature. Preferably, before use as a vaccine the membrane virus target particles and membrane virus fusion particles are fixed. Fixation can be carried out as described by LaCasse et al (1) i.e. with formaldehyde. For a vaccine, target and fusion particles separately produced in different cells can be mixed at appropriate ratio, e.g. (1:1), incubated for a suitable time, e.g. 90 min at 37°C, purified e.g. by ultracentrifugation and fixed e.g. with formaldehyde.

### Brief description of the Figures

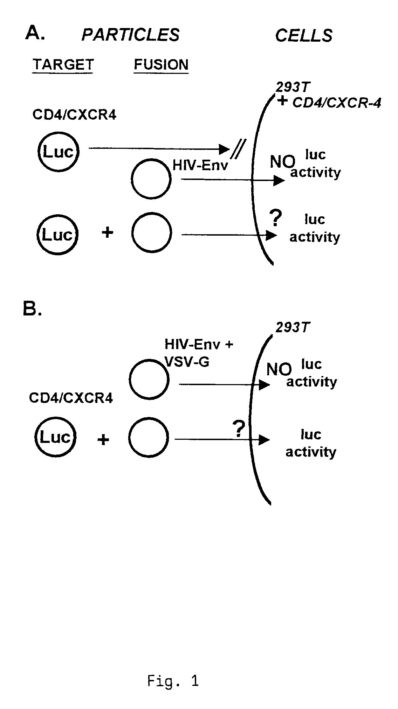

### Figure 1. Strategies to analyse inter-membrane virus particle fusion and the infectivities of fused (fusing) viral structures.

A. Target particles (left), generated as described in Example 1, carry a genome encoding a marker luciferase gene (Luc) and have incorporated CD4 and CXCR4, respectively. Fusion particles (middle) lack marker gene and have incorporated HIV-Env. Target particles, fusion particles or mixtures of target particles and fusion particles were analysed as to their ability to transmit luciferase (luc) activity to indicator CD4/CXCR4-expressing 293T cells.
**B.** As in A. but fusion particles have incorporated VSV-G in addition to HIV-Env. The indicator cells are 293T cells, not expressing CD4/CXCR4.

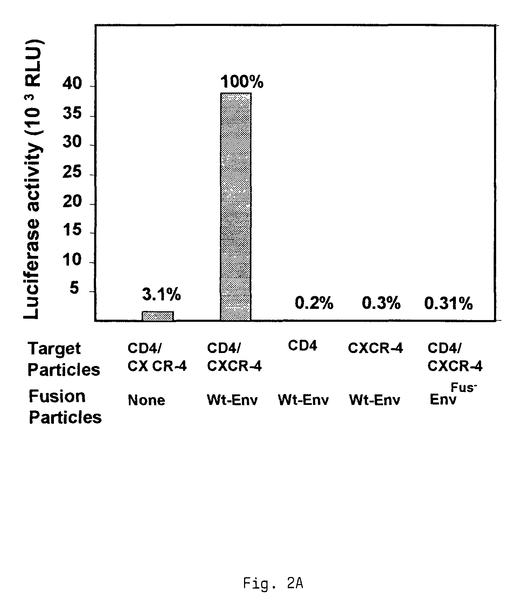

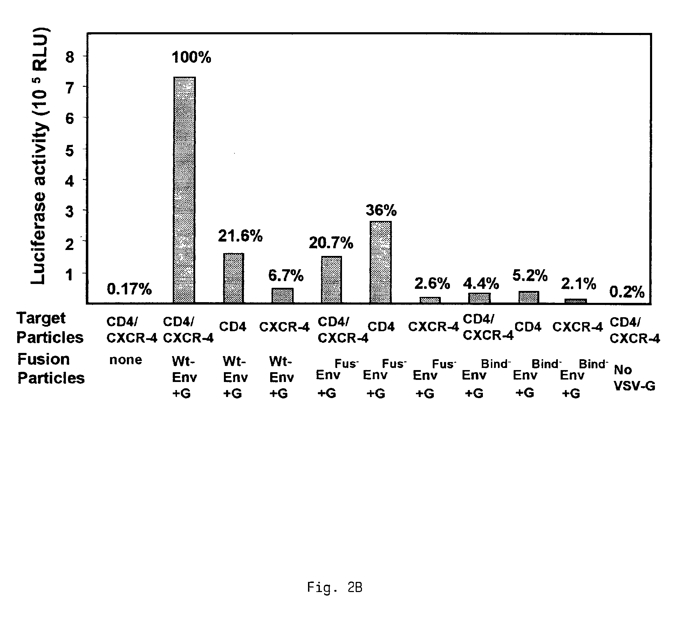

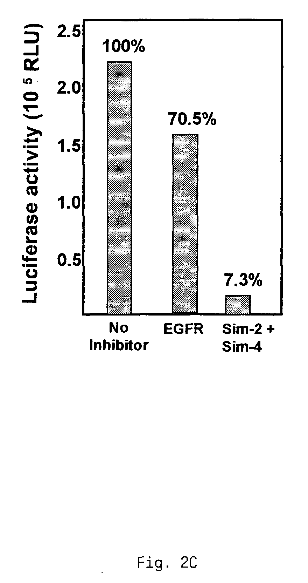

### Figure 2. Luciferase gene transduction after inter-viral particle fusion mediated by CD4/CXCR4 and HIV-Env.

A. HIV-Env mediated gene transduction. The glycoprotein compositions of the respective target particles and fusion particles, which were premixed prior to application to CD4/CXCR4 expressing 293T cells, and the resulting luciferase activities (total activities per culture given on the Y-axis) in lysates of transduced cells are given for a representive experiment (from 3 independant experiments). The numbers above each block give the percentage luciferase activity in comparison to that generated by a mixture of CD4/CXCR4-target particles and Wt-Env-fusion particles.
B. VSV-G mediated gene transduction. The glycoprotein compositions of the respective target particles and fusion particles, which were premixed prior to application to 293T cells (not expressing CD4/CXCR4) and the resulting luciferase activities (total activities per culture given on the Y-axis) in lysates of transduced 293T cells are given for a representative experiment. G denotes VSV-G glycoprotein. The numbers above each block give the percentage luciferase activity in comparison to that generated by a mixture of CD4/CXCR4 target particles and Wt-Env/G-fusion particles.
C. Inhibition of transduction by monoclonal antibody against CD4. Mixtures of CD4/CXCR4 target particles and Wt-Env/G-fusion particles were either untreated (no inhibitor), preincubated with irrelevant antibody (EGFR) or with antibody against CD4 (SIM-2 + SIM-4). The numbers above each block give the percentage luciferase activity in comparison to that of the untreated sample.

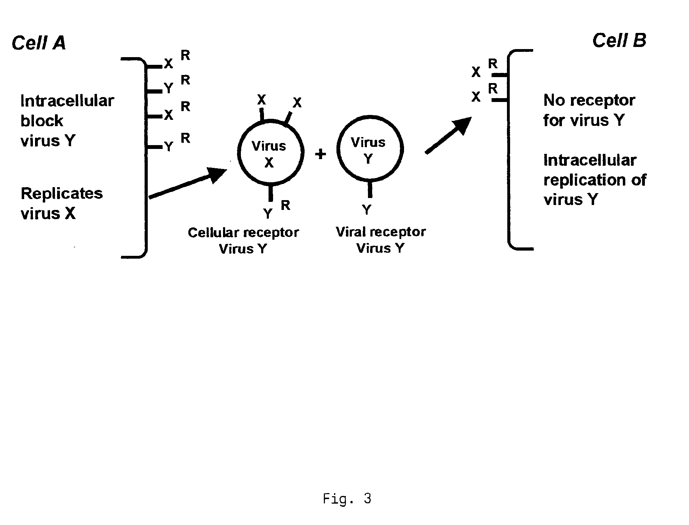

### Figure 3. Targeting of viral genome to cells lacking cellular receptor as a result of specific inter-viral fusion.

The hypothetical producer cell, A, for virus X (left) carries cellular receptors for virus X and Y (X^{R} and Y^{R}, respectively) but (in order to distinguish this phenomenon from pseudotyping) is unable to replicate virus Y. Released virus X, with incorporated Y^{R}, can fuse with virus Y, produced elsewhere in the infected organism. The resultant fused viral structure is able to transfer the genome of virus Y, via viral receptor X, to a novel target cell (right) lacking Y^{R} but carrying X^{R}.

### Example 1: General procedures

### (A) Constructs employed

PNL4-3.Luc.R-E-, an env-defective HIV-1 particle-encoding construct, encoding luciferase instead of nef (2); (Chen et al., J. Virol. 68, (1994), 654-660), was used to generate target particles. PSG5-CD4-Gpl was a kind gift from Marc Alizon, INSERM, Paris. It encodes GPI-anchored human CD4 (referred herein as CD4) with the extracellular domain of CD4 and the C-terminal region of CD55 (method for generation: Ho et al., Gene 77, (1989), 51-59; Horton et al., Gene 77, (1989), 61-68). It was employed since this construct (plus CXCR4) results in 2-3 times better infection of HIV-Env expressing cells than Wt-CD4 (data not shown). Expression of CXCR4 was achieved employing pc.Fusin (9) (for target particles) or pBABE.Fusin (9) (to generate CD4/CXCR4 expressing 293T cells, see below). pc.Fusin and pBABE.Fusin were obtained from the AIDS Research and Reference Reagent Program. NIH, NIAID. pc is pcDNA/amp (expression vector from InVitrogen) and pBABE-puro is described in Morgenstern and Land, Nucl. Acids Res. 18; 3587-3596 (1990). The particle-encoding construct, pNL4-3ΔΨΔEnv3, based on pNL4-3 (10), contains a frame-shift mutation at the beginning of the env gene (11) and a deletion in the packaging signal (12). It was employed to generate fusion particles. PLßAcenv/neo (referred to here as pl-HIV-Env) encodes Wt-HIV-Env (13). The derivatives referred to here as HIV-Env^{Fus-} and HIV-Env^{Bind-} carry mutations within the fusion peptide (gp41.2) (14) and CD4 binding site (15), respectively. PEx-VSV-G encodes the vesicular stomatitis virus (VSV) G glycoprotein. (gene sequence: Rose and Bergmann, Cell 34, (1983), 513-524).

### (B) Generation of target and fusion particles

PNL4-3.Luc.R-E-, plus PSG5-CD4-Gpl and pc.Fusin singly and in combination (to generate target particles) or pNL4-3ΔΨEnv3 plus Wt or mutated versions of pHIV-Env in the presence or absence of pEx-VSV-G (to generate fusion particles) were cotransfected into 293T cells using standard calcium phosphate procedures. 48 hours post-transfection, the amounts of released particles were quantitated by ELISA for HIV-1-CA (Innogenetics, Belgium. Expression of CD4, CXCR4 (employing CXCR4 monoclonal antibody (12G5) (16), HIV-Env and VSV-G (antiserum from Lee Biomolecular, San Diego, were confirmed by indirect immunofluorescence analyses employing respective specific antisera as previously described (4, 13)(data not shown).

### (C) Particle fusion

Appropriate volumes of culture supernatants, filtered through a 0.45 µm filter, and representing equal amounts of different target particles on the one hand, and equal amounts of different fusion particles on the other hand, were adjusted to 8 µM polybrene , mixed (at a ratio of 1: 1) in a final volume of 2 ml and incubated for 90 min at 37°C, 5%CO², before application to subconfluent cultures of fresh indicator cells (either 293T cells or CD4/CXCR4 expressing 293T cells) in a 12 well (4.9 cm²) multi-dish. CD4/CXCR4 expressing 293T indicator cells were generated by stable expression of CD4 (from pIGRE-CD4 (4)). plus CXCR4 (from pBABE.fusin (9)) in 293T cells. 72h later, cell lysates were prepared and total luciferase activities per culture measured using standard procedures.

### (D) Inhibition of particle fusion

Culture supernatants from SIM-2 plus SIM-4 (1:1) hybridoma cells (17), releasing antibodies reacting with CD4 and able to inhibit infection by pNL4-3 HIV, or from H-EGFR-RI hybridoma cells (18), releasing antibodies against human epidermal growth factor receptor (EGFR) which are thus irrelevant in this context, were added to the mixtures of target particles and fusion particles during the incubation period before application to indicator cells. The final concentration of mouse antibody was approx. 1 µg/ml.

### Example 2: Membrane fusion between HIV-like particles

The first strategy which was employed to analyse membrane fusion between HIV-like particles is illustrated in Fig. IA. Particles, with incorporated CD4 and CXCR4 and carrying a luciferase reporter gene, are referred to as CD4/CXCR4 target particles. Particles with incorporated Wt-HIV-Env, but lacking transducible vector, are referred to as Wt-Env-fusion particles. It was hypothesised that if membrane fusion between target and fusion particles would occur, the resultant fused (fusing)viral structures could be infectious for and be able to transduce the luciferase gene to CD4/CXCR4 expressing 293T cells. Target particles were generated by cotransfecting 293T cells with an Env-defective proviral construct, pNL4-3.Luc.R-E- (2) encoding the luciferase gene instead of nef, plus expression vectors for CD4 and CXCR4, either alone or in combination. It has previously been shown (3) that CD4 and CXCR4 can be incorporated into HIV particles and mediate infectivity into HIV-1 infected cells (3) and it was confirmed that the CD4/CXCR4 target particles were infectious for, and efficiently transmitted luciferase activity into HIV-Env expressing BJAB-Envi-cells (4) (data not shown). In the strategy shown in Fig. 1 A, Wt-Env fusion particles or mutants thereof were generated by coexpressing an Env-defective particle-encoding plasmid with a deletion in the packaging signal, with Wt or mutant HIV-Env, respectively. Appropriate volumes of culture supernatants, representing equal amounts of target particles on the one hand and equal amounts of fusion particles on the other hand, were mixed (at a ratio of 1: 1) and preincubated for 90 min. at 37°C to allow potential fusion to occur (see Example 1). Mixed supernatants were subsequently applied to CD4/CXCR4 expressing 293T cells and 3 days post-transduction, the luciferase activities in cell lysates were determined. As shown in Fig. 2A, CD4/CXCR4 target particles alone resulted in only very low levels of luciferase activity after transfer to CD4/CXCR4 expressing 293T cells. However, when these CD4/CXCR4 target particles were mixed with culture supernatants containing Wt-Env fusion particles, the resultant mixture led to a 30-50 fold increase in transduction of CD4/CXCR4 expressing 293T cells. The luciferase values obtained were, in fact, about 2% of those obtained when Wt-HIV-Env itself, instead of CD4/CXCR4, was directly incorporated into target particles (not shown). This value of 2% is relatively high. This is because, on the one hand, the particle concentration in the culture supernatants is in the range of only 1 - 5 µg/ml making it unlikely that every target particle can fuse with a fusion particle. Additionally, it is unlikely that fused viral structures are as infectious as genuine viral particles. Several controls were performed in order to confirm that the observed transduction was due to a fusion event (and not just a binding event) requiring all components namely CD4, CXCR4 and Wt-HIV-Env in a functional form. On the one hand, CD4 target particles or CXCR4 target particles, with incorporated CD4 or CXCR4 alone, respectively, were mixed with Wt-Env fusion particles. On the other hand, Enf^{Fus-}-fusion particles, with incorporated non-fusogenic HIV-Env with a mutation in the gp41 fusion peptide, were mixed with CD4/CXCR4 target particles. All these mixtures gave only background levels of transduction. These results clearly indicate that specific CD4/CXCR4/HIV-Env-dependant inter-membrane virus fusion can occur and that the resultant fused particles are infectious.

### Example 3: Enhanced infectivity by using fusion particles having incorporated the VSV-G glycoprotein

When initially the experimental strategy illustrated in Fig. 1A was developed, the possibility was considered that the potential infection by fused (fusing) particles could be an inefficient process, and thus difficult to measure, since a fraction of the HIV-Env would already have engaged in membrane fusion with the target particles and, thus, would no longer be available to mediate infection of cells. Although, as shown in Fig. 2A, this consideration was unwarrented, concomitantly the alternative strategy illustrated in Fig. IB was developed which would allow to circumvent this potential problem. In this strategy, in addition to HIV-Env, the fusion particles (now referred to as Wt-Env/G fusion particles) have incorporated VSV-G glycoprotein, which is a potent mediator of membrane virus vector transduction (5, 6). In this situation, after potential fusion between target particles and fusion particles, which should still be mediated by CD4/CXCR4 and HIV-Env, the infection of 293T cells (lacking CD4/CXCR4) and transduction of the luciferase gene by the fused (fusing) particles should be mediated by VSV-G. As shown in Fig. 2B, this is in fact the case and mixtures of CD4/CXCR4 target particles and Wt-Env/G fusion particles resulted in high luciferase activity in transduced 293T cell lysates. The VSV-G mediated luciferase activities were in fact 20-50 times higher than those obtained with HIV-Env (Fig. 2A), consistant with the superior ability of VSV-G to mediate membrane virus transduction as described by others (5, 6). It was initially a surprising observation that mixtures of Wt-Env/G fusion particles, or fusion Env^{Fus-}/G particles, having incorporated HIV-Env mutated within the gp41 fusion peptide, plus CD4-target particles having incorporated CD4 alone, still led to significant transduction of luciferase (Fig. 2B). The values obtained in several different experiments were in the range of 20-50% of the values obtained with CD4/CXCR4 target particles plus Wt-Env/G fusion particles. In fact, all further mixtures of target particles and fusion particles (CXCR4 target particles with CXCR4 alone, Env^{Bind-}/G-fusion particles with defective HIV-Env mutated at the CD4 binding site (Fig. 2B), or VSV-G alone infusion particles (not shown) also resulted in luciferase transduction as long as the fusion particles contained VSV-G glycoprotein. In these latter cases, the luciferase values obtained were in the range of 2-10% of the values obtained with CD4/CXCR4 target particles plus Wt-Env/G-fusion particies (Fig. 2B). On the other hand, when VSV-G was absent from fusion particles, and these were mixed with CD4/CXCR4 target particles, only background levels of luciferase transduction into 293T cells were observed. These positive but clearly reduced luciferase transduction values, in the absence of CD4/CXCR4/HIV-Env-mediated fusion, but in the presence of VSV-G, are assumed to be the result of particle fusion mediated by VSV-G on fusion particles and VSV-G cellular receptor which has been incorporated into target particles. This would be despite the fact that VSV-G normally requires low pH-value in the cellular endosome, after receptor-mediated uptake, to trigger membrane fusion. The VSV-G cellular receptor has been shown to include phospholipid components (7,8) which would also be incorporated into virus-like particles. In those situations in which CD4 and HIV-Env can bind to each other but not fuse (CD4-target particles, Env^{Fus-}G-fusion particles), the luciferase transduction values of mixed particles is significantly higher (20-50% of the values obtained with CD4/CXCR4-target plus Wt-Env/G-fusion particles) than in the absence of CD4/HIV-Env binding (2-10%). This is presumably due to the binding between CD4 and HIV-Env leading to a closer proximity between target particles and fusion particles and allowing more efficient membrane fusion mediated by VSV-G and its cellular receptor. Lastly, as a further proof of the role of CD4 in mediating binding and fusion between particles, the presence of CD4 monoclonal antibody during the incubation between CD4/CXCR4 target particles and Wt-Env/G-fusion particles very significantly inhibited luciferase transduction (Fig. 2C).

Conclusions: The results of these experiments confirm, on the one hand, the results obtained using the first strategy (Fig. 1A, 2A), namely that specific CD4/CXCR4/HIV-Env-dependant inter-membrane virus fusion can occur and that the resultant fused (fusing) particles are infectious. The results, however, further indicate that, even in the absence of overexpression, cellular receptors for membrane viruses (VSV as an example) can be incorporated into foreign virus particles in amounts sufficient to mediate inter-particle fusion with virus particles carrying the respective viral receptor. Thus, depending on the nature of the membrane virus, the tissue distribution of its cellular receptor and the availability of this molecule for incorporation into particles, interviral membrane fusion may be a previously unrecognized in vivo process (not achievable by simple pseudotyping) by which the genomes of membrane viruses can target cells lacking an appropriate cellular receptor. A theoretical scenario describing such a situation is shown in Fig. 3. In this situation, membrane virus X, released from cells carrying a cellular receptor for both virus X and Y (but intracellularly blocked for replication of virus Y) would incorporate the cellular receptor for virus Y (Y^{R}) and thus fuse with virus Y itself (produced in permissive cells). The resulting fused (fusing) viral structures would then be able to transfer the genome of virus Y to further cells lacking Y^{R} but carrying the cellular receptor for virus X (X^{R}). Depending on the viruses and target cells involved, this could have far-reaching consequences for the infected organism, e. g. stable production of virus Y within the mammalian from a hithertoo unrecognized cell reservoir.

### List of References

1. LaCasse et al. Fusion-competent vaccines: broad neutralisation of primary isolates of HIV. Science 283, 357-362 (1999).
2. He et al. Human immunodeficiency virus type 1 viral protein R (Vpr) arrests cells in the G2 phase of the cell cycle by inhibiting p34^{cdc2} activity. J. Virol. 69, 6705-6711 (1995).
3. Endres et al. Targeting of HIV- and SIV-infected cells by CD4-chemokine receptor pseudotypes. Science 278, 1462-1464 (1997).
4. Krüger et al. Generation of lymphocyte cell lines coexpressing CD4 and wild-type or mutant HIV type 1 glycoproteins: implications for HIV type 1 Env-induced cell lysis. Aids Res. & Human Retroviruses 12, 783-790 (1996).
5. Akkina et al. High efficiency gene transfer into CD34+ cells with a human immunodeficiency virus type 1-based membrane virus vector pseudotyped with vesicular stomatitis virus envelope glycoprotein G. J. Virol. 70, 2581-2585 (1996).
6. Aiken. Pseudotyping human immunodeficiency virus type 1 (HIV-1) by the glycoprotein of vesicular stomatitis virus targets HIV-1 entry to an endocytic pathway and suppresses both the requirement for nef and the sensitivity to cyclosporin A. J. Virol. 71, 5871-5877 (1997).
7. Schlegel et al. Inhibition of VSV binding and infectivity by phosphatidylserine: is phosphatidylserine a VSV-binding site? Cell 32, 639-646 (1983).
8. Mastromarino et al. Characterisation of membrane components of the erythrocyte involved in vesicular stomatitis virus attachment and fusion at acidic pH. J. Gen. Virol. 68, 2359-2369 (1987).
9. Deng et al. Identification of a major co-receptorfor primaryisolates of HIV-1. Nature 381, 661-666 (1996).
10. Adachi et al. Production of acquired immunodefiency syndrome-associated retrovirus in human and non-human cells transfected with an infectious molecular clone. J. Virol. 59, 284-291 (1986).
11. Henriksson and Bosch. Inhibition of cellular glycoprotein incorporation into human immunodeficiency virus-like particles by coexpression of additional cellular interaction partner. Virology 251, 16-21 (1998).
12. Corbeau et al. Efficient gene transfer by a human immunodeficiency virus type 1 (HIV-1)-derived vector utilizing a stable HIV packaging cell line. Proc. Natl. Acad. Sci. USA 93, 14070-14075(1996).
13. Kräusslich et al. Analysis of protein expression and virus-like particle formation in mammalian cells lines stably expressing HIV-1 gag and env gene products with or without active HIV proteinase. Virology 192, 605-617 (1993).
14. Freed et al. Characterisation of the fusion domain of the human immunodeficiency virus type 1 envelope glycoprotein gp41. Proc. Natl. Acad. Sci. USA 87, 4650-4654 (1990).
15. Lasky et al. Delineation of a region of the human immunodeficiency virus type 1 gp 120 glycoprotein critical for interaction with the CD4 receptor. Cell 50, 975-985 (1987).
16. Endres et al. CD4-independant infection by HIV-2 is mediated by fusin/CXCR4. Cell 87, 745-756 (1996).
17. McCallus et al. Construction of a recombinant bacterial human CD4 expression system producing a bioactive CD4 molecule. Viral Immunol. 5, 163-172 (1992).
18. Waterfield et al. a monoclonal antibody to the epidermal growth factor receptor. J. Cell. Biochem. 20, 149-161 (1982).

## Claims

1. A composition of membrane virus subviral particles, comprising
(a) an env-defective, at least one cellular receptor and at least one coreceptor containing membrane virus target particle encoded by an env-defective membrane virus particle-encoding vector construct, at least one cellular receptor encoding vector(s) and at least one coreceptor encoding vector(s); and
(b) a membrane virus fusion particle encoded by an env-defective membrane virus particle-encoding vector construct and an env-encoding vector,
wherein said composition of membrane virus subviral particles is capable of inter-membrane virus particle membrane fusion resulting in the formation of fusing membrane virus particles.

2. The composition of claim 1, wherein the inter-membrane virus particle membrane fusion results in fused membrane virus particles.

3. The composition of claim 1 or 2, wherein the membrane virus subviral particles are retrovirus-like particles.

4. The composition of claim 3, wherein the retrovirus-like particles are HIV-like particles.

5. The composition of claim 4, wherein the HIV-like particles contain CD4 as cellular receptor.

6. The composition of claim 4 or 5, wherein the HIV-like particles contain CXCR4 as coreceptor.

7. The composition of any one of claims 4 to 6, wherein the env-encoding vector for the membrane virus fusion particle of (b) encodes the viral receptor glycoprotein of HIV-1.

8. The composition of any one of claims 4 to 7, wherein in the env-defective membrane virus particle encoding vector construct for the target particle of (a) the gene encoding nef is inactivated.

9. A method of producing a membrane virus target particle or a membrane virus fusion particle, comprising
(a) cotransfecting a mammalian host cell with the vectors as defined in any one of claims 1(a) or 1(b) to 8 under conditions such that the proteins encoded by said vectors are expressed; and
(b) recovering the membrane virus target particle or membrane virus fusion particle from the culture supernatant.

10. A vaccine comprising a therapeutically effective amount of the composition of any one of claims 1 to 8 or the composition produced according to the method of claim 9.

11. Use of the composition of any one of claims 1 to 8 or the composition produced according to the method of claim 9 for the preparation of a prophylactical or therapeutical vaccine.
